# EUROPEAN PATENT APPLICATION

(11) **EP 2 351 537 A1**
(43) Date of publication of application: **03.08.2011**
(21) Application number: 09826209.0
(22) Date of filing: 01.04.2009
(51) Int. Cl.: A61C 8/02

(54) **DENTAL DRILL FOR FLAPLESS IMPLANT SURGERY**

(30) Priority: 13.11.2008 KR 20080112555
(71) Applicant: Megagen Implant Co., Ltd., Gyeongsangbuk-do 712-852 (KR)
(72) Inventor: CHOI, Byeong Ho, Wonju-si Gangwon-do 220-958 (KR); JEONG, Seung Mi, Wonju-si Gangwon-do 220-734 (KR)
(74) Representative: Grünecker, Kinkeldey, Stockmair & Schwanhäusser Anwaltssozietät
(86) International application number: PCT/KR2009/001664
(87) International publication number: WO 2010/055976

(57) **Abstract**

A dental drill is intended for performing flapless implant surgery by being mounted on a drill device. The dental drill comprises a drill body and a mounting portion for mounting the drill body to a drill device. The drill body comprises: a cutting part for providing a side blade having a certain length from the cutting edge, and a body portion with limited cutting ability. Tooth bone can be easily removed because of the cutting part at the end portion of the drill body. Also, gums can be protected because the body portion is not only formed with the cutting part integrally, but the side blade either does not exist or is removed.

## Description

### Technical Field

The present invention relates to a flapless implant, and more particularly, to a dental drill for flapless implant surgery, which can effectively protect gums at the flapless implant surgery and minimize aftereffects after the surgery.

### Background Art

Implant surgery broadly includes flap implant surgery and flapless implant surgery.

In the flap implant surgery, gums are widely cut before implanting a fixture into an alveolar bone, the alveolar bone is exposed to the outside by widely opening out a flap having periosteum and a gum tissue, and osseous resection is carried out by a drill to prepare a cavity for implanting the fixture. The fixture is implanted into the cavity, and then the flap is closed and sutured, thereby primarily completing implant surgery.

In the flap implant surgery, osteomucosal incision is made when implanting the fixture, and the flap is elevated to expose the bone. Generally, to secure a proper view and facilitate approach to a surgery part, the bone is so widely exposed that a swelling, a pain, infection or the like aftereffects may come out, and damage of the periosteum may cause bone resorption around the fixture and lower a synostotic efficiency. Therefore, more chances of successful clinical trial are given when the flap is not formed than when the flap is formed.

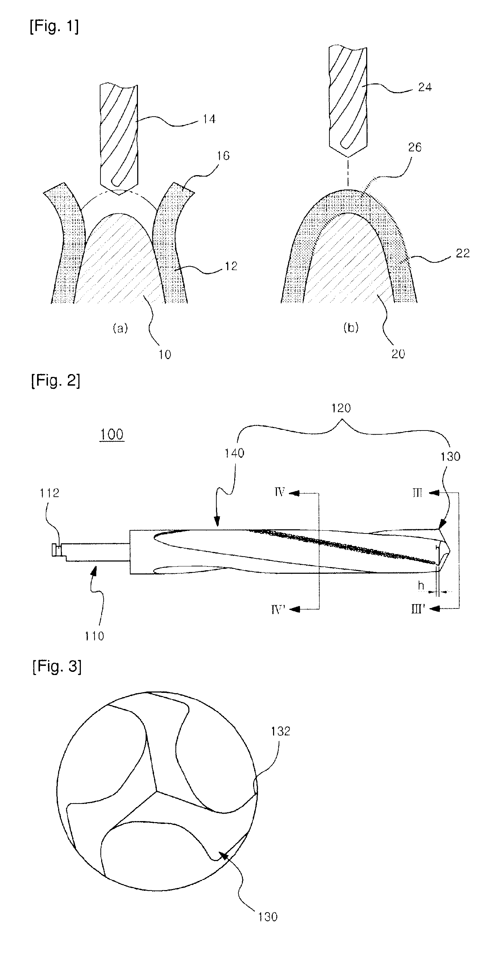

FIG. 1 is a cross-section view for explaining a conventional flap implant surgery and flapless implant surgery.

Referring to (a) of FIG. 1, gums 12 are cut and widely open to expose an alveolar bone 10, and a cavity is formed on the exposed alveolar bone 10 by a drill 14.

As described above, if a flap 16 is widely formed, a swelling, infection or the like may arise due to the invasion of germs even though the flap 16 is closed and sutured with a surgical suture. Also, a damaged part is so large that severe pain can be caused after the surgery.

Referring to (b) of FIG. 1, instead of cutting the gums 22, a hole 26 is made in the gums 22 largely more than a diameter of a drill so that it can easily receive the drill, thereby exposing an alveolar bone 20. The drill 24 carries out the osseous resection while approaching the formed hole 26 and thus forms a cavity in the alveolar bone 20. Specifically, in the flapless implant surgery, a cylindrical hole 26 is formed by removing the gums as large as a diameter of about 4-6mm, and the drill having a diameter 4∼5mm or less is inserted in the hole 26, so that the osseous resection can be carried out and then the fixture can be implanted.

The flapless implant surgery refers to a method in which the osseous resection is carried out by passing the drill through the gums in the state that the alveolar bone is not exposed, i.e., the alveolar bone is covered with the gums, and the fixture is implanted in the alveolar bone through the formed cavity. With recent development of computerized tomography (CT) and computer programming, the flapless implant surgery is more accurately performed since the shape of the alveolar bone can be exactly grasped without exposing a bone surface.

When performing the flapless implant surgery, the existing implant drill having a long blade at its lateral side cuts the gums while carrying out the osseous resection, so that it cannot protect the gums. In a particular case if the thickness of the gums is 1∼2mm and the length of the lateral blade is 2mm or more, the gums may be cut at the overall height thereof and damaged.

If the gums are cut by the drill, it is difficult to cover a top of the fixture with the gums after the flapless implant surgery, and a space between an implant surface and the gums becomes widen to thereby delay a cure of the gums and facilitate the infection. Thus, the gums around the implant may get an infection, and osseointegration between the fixture and the alveolar bone may be deteriorated as the alveolar bone is absorbed.

### Disclosure of Invention

### Technical Problem

The present invention provides a dental drill capable of minimizing damage of gums during flapless implant surgery.

The present invention provides a dental drill capable of performing stable drilling even while removing an alveolar bone.

### Technical solution

According to an exemplary embodiment of the present invention, a dental drill is mounted to a drill device and carries out flapless implant surgery, which includes a drill body; and a mounting part for mounting the drill body to the drill device, the drill body including a cutting part formed with a lateral blade from an end to a depth corresponding to a predetermined length, and a body part connected integrally with the cutting part and having limited cutting ability. The cutting part is provided at the end part of the drill body, so that osseous resection can be performed without difficulty. Further, the body part is not only formed integrally with the cutting part but also has no or removed lateral blade, so that gums can be protected.

The length of the cutting part may be 0.5mm or less. In the body part, the other parts of the lateral blades are cut or processed to be rounded.

When carrying out the flapless implant surgery, only a small hole is formed in the gums, so that a bone can be removed by inserting the drill in between the gums, thereby carrying out the osseous resection while protecting the gums when the fixture is implanted.

### Advantageous Effects

According to an exemplary embodiment of the present invention, the implant is inserted on the basis of the flapless implant surgery, the fixture can be completely buried under the gums, the gums can be safely protected while the drill operates, the fixture is implanted with close contact between the surface of the implant and the gums on the basis of the flapless implant surgery, and it is possible to minimize aftereffects that may occur in the gums after the flapless implant surgery.

### Brief Description of the Drawings

FIG. 1 is a cross-section view for explaining a conventional flap implant surgery and flapless implant surgery.
FIG. 2 is a lateral view for explaining a dental drill according to an exemplary embodiment of the present invention.
FIG. 3 is a front view taken along line III-III' in FIG. 2.
FIG. 4 is a cross-section view taken along line IV-IV in FIG. 2.
FIGs. 5 and 6 are cross-section views for explaining use of the dental drill according to an exemplary embodiment of the present invention.

### Best Mode for Carrying Out the Invention

Exemplary embodiments of the present invention will be described with reference to accompanying drawings, but the present invention is not limited to the exemplary embodiment.

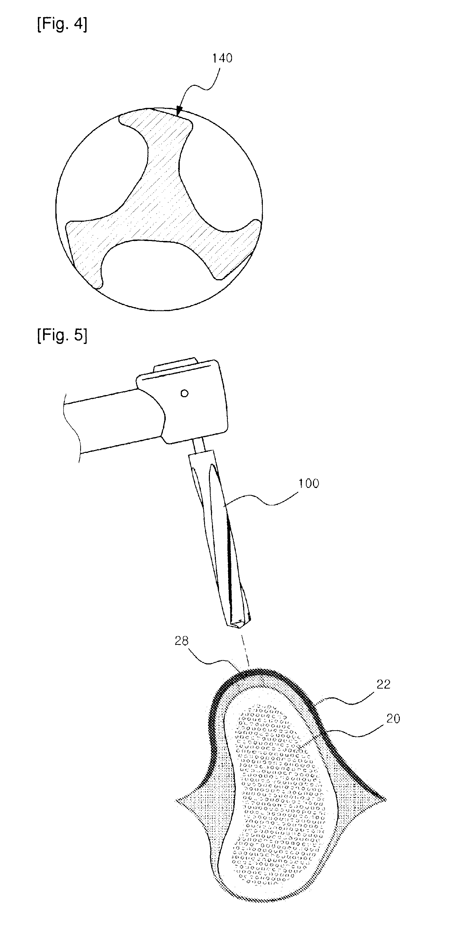

FIG. 2 is a lateral view for explaining a dental drill according to an exemplary embodiment of the present invention, FIG. 3 is a front view taken along line III-III' in FIG. 2. FIG. 4 is a cross-section view taken along line IV-IV' in FIG. 2.

Referring to FIG. 2, a dental drill 100 includes a drill body 120, and a mounting part 110. The drill body 120 includes a cutting part 130 at an end thereof, and a body part 140 formed integrally with the cutting part 130 therein. While the cutting part 130 is formed with a blade 132 at a lateral side and has cutting ability, the body part 140 has no cutting ability since a separate blade is not provided or is removed.

The drill body 120 can be mounted to a drill device through the mounting part 110. The mounting part 110 may be formed to have the same shape and size as a drill mounting part of the drill device regardless of a diameter of a drill. The mounting part 110 may have a smaller diameter than the drill body 120, and be formed with a fastening groove 112 formed along a circumference of an inner end part thereof. Further, the end part of the mounting part 110 is approximately shaped like a D-cut and rotatable together with the drill device.

The drill body 120 may be formed as a general drill shape, and include two or three lateral blades. The drill body 120 includes the cutting part 130 effectively formed with the lateral blades 132, and the body part 140 having no cutting ability. The cutting part 130 formed with the lateral blades is formed at an end part and used for resecting the alveolar bone. The body part 140 not only connects integrally with the cutting part 130 but also guides a moving direction of the drill with its outer surface supported on an external hole or a tissue. To this end, the diameter of the body part 140 may be equal to or a little smaller than the diameter of the cutting part 120. In a flapless case, it is impossible to remove gums, so that heat due to drilling cannot be easily discharged. Therefore, the heat can be easily discharged by making the diameter of the body part 140 small.

According to this exemplary embodiment, the cutting part 130 formed with the lateral blades may have a length h of about 0.5mm or less. If the cutting part 130 is longer than 0.5mm, it may cause a tissue of gums to be damaged. On the other hand, if the cutting part 130 is too short, it cannot remove the alveolar bone or the like tissue as well as the gums.

Referring to FIG. 3, the lateral blades 132 is formed at an end part of the cutting part 130, and the cutting part 130 having a length of 0.5mm or less is enough for cutting.

On the other hand, referring to FIG. 4, a part of the body part 140, where the lateral blades have to be formed, is rounded and thus has no cutting ability. However, as described above, the body part 140, together with the cutting part 130, forms a cavity. Therefore, the diameter of the body part 140 has to be equal to or at least a little larger than that of the cutting part 130. This is because the body part 140 may be supported on an inner wall of the cavity while forming the cavity, and serves to guide the cutting part 130 to move straightly along a predetermined course.

Below, a process of forming a cavity using a dental drill according to an exemplary embodiment of the present invention will be described.

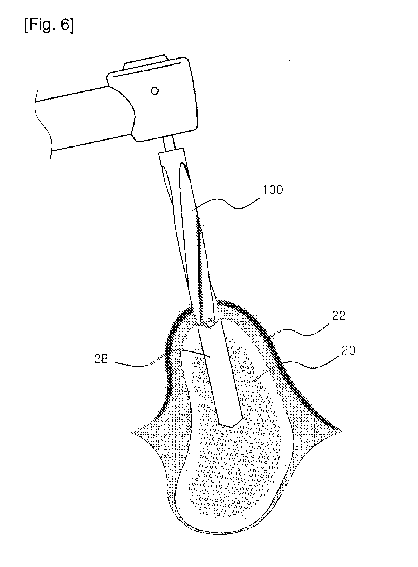

FIGs. 5 and 6 are cross-section views for explaining use of the dental drill according to an exemplary embodiment of the present invention.

Referring to FIG. 5, the gums 22 are partially removed to form a hole 28, so that a part of the alveolar bone 20 can be exposed to the outside through the hole 28. As mentioned above, the drill 100 having the lateral blades at only the cutting edge thereof can approach the alveolar bone 20 through the hole 28, and thus the drill 100 can form the cavity for implanting a fixture.

In this exemplary embodiment, the drill 100 having a diameter smaller than the fixture is used in consideration of the diameter of the fixture to be used. Also, the cavity may be designed to have a smaller diameter than the fixture to be used since the fixture is inserted in the alveolar bone through the cavity. Further, the drill having a smaller diameter than the fixture may be selected in accordance with the diameter of the cavity.

Generally, the conventional fixtures having diameters of about 3.5mm, 4.0mm, 4.5mm, 5.0mm, and so on have been used, and therefore the drills having diameters of about 3.0mm, 3.5mm, 4.0mm, 4.5mm, and so on may be selected, respectively.

According to the present exemplary embodiment, the dental drill 100 has the cutting ability from its end to a depth of about 0.5mm, but has no cutting ability thereafter, so that the gums can be little damaged and the diameter of the hole 28 can be smaller than that of the drill 100.

Referring to FIG. 6, the drill 100 passes through the gums 22 through the hole 28 and forms a hole or a cavity 21 in the alveolar bone 20. As described above, in the drill 100 with the cutting part and the body part according to this exemplary embodiment, the diameter of the hole 28 may be smaller than that of the drill 100. If the hole 28 has such a small diameter, the surface of the fixture can be in close contact with the tissue of the gums when the fixture is implanted after the drilling, and excellent and close contact between the gums and a heating abutment is expected when the healing abutment is mounted.

However, if the hole 28 is too small, the gums may be damaged by the body part 140 during the drilling. Further, if the hole 28 has an excessively smaller diameter than the drill, the close contact between the fixture and the gums may be disturbed. Accordingly, the size of the hole 28 may be selected to be smaller than the diameter of the drill 100 by about 0.4mm∼1.0mm. In other words, the size of the hole 28 may be determined to be smaller than the diameter of the fixture by about 1.0mm∼1.5mm.

After such flapless implantation, the fixture can be completely buried under the gums before secondary surgery for prosthesis, during which the alveolar bone and the fixture can be more firmly integrated with each other, thereby improving osseointegration.

Also, the flapless implant surgery, difficult for the existing implant drill, can be realized, and a curing time can be reduced since it is possible to bury the fixture under the gums.

## Claims

1. A dental drill mounted to a drill device and carrying out flapless implant surgery, the dental drill comprising:
a drill body; and
a mounting part for mounting the drill body to the drill device,
the drill body comprising a cutting part formed with a lateral blade from an end to a depth corresponding to a predetermined length, and a body part connected integrally with the cutting part and having limited cutting ability.

2. The dental drill according to claim 1, wherein the length of the cutting part is 0.5mm or less.

3. The dental drill according to claim 1, wherein the body part comprises no lateral blade.

4. The dental drill according to claim 1, wherein the body part is formed to have the same diameter as the cutting part.

5. The dental drill according to claim 1, wherein the body part is formed to have a little smaller diameter than the cutting part.
